(19) Europäisches Patentamt
European Patent Office
Office européen des brevets

(11) EP 1 292 599 B1

(12) **EUROPÄISCHE PATENTSCHRIFT**

(45) Veröffentlichungstag und Bekanntmachung des Hinweises auf die Patenterteilung:
**24.03.2004 Patentblatt 2004/13**

(21) Anmeldenummer: **01945142.6**

(22) Anmeldetag: **17.05.2001**

(51) Int Cl.[7]: **C07F 1/02**

(86) Internationale Anmeldenummer:
**PCT/EP2001/005609**

(87) Internationale Veröffentlichungsnummer:
**WO 2001/094354 (13.12.2001 Gazette 2001/50)**

(54) **LÖSUNGEN VON LITHIUM-PYRROLIDIN IN THF/KOHLENWASSERSTOFFEN**

SOLUTIONS OF LITHIUM PYRROLIDINE IN TETRAHYDROFURAN (THF)/HYDROCARBONS

SOLUTIONS DE LITHIUM-PYRROLIDINE DANS UN MELANGE DE TETRAHYDROFURANE/HYDROCARBURES

(84) Benannte Vertragsstaaten:
**AT BE CH CY DE DK ES FI FR GB GR IE IT LI LU MC NL PT SE TR**

(30) Priorität: **07.06.2000 DE 10027604**

(43) Veröffentlichungstag der Anmeldung:
**19.03.2003 Patentblatt 2003/12**

(73) Patentinhaber: **Chemetall GmbH
60487 Frankfurt (DE)**

(72) Erfinder:
- **WEISS, Wilfried
37632 Eschershausen (DE)**
- **EMMEL, Ute
65929 Frankfurt am Main (DE)**
- **TOTTER, Franz
63110 Rodgau (DE)**
- **WIETELMANN, Ulrich
61381 Friedrichsdorf (DE)**

(74) Vertreter: **Uppena, Franz, Dr.
Dynamit Nobel Aktiengesellschaft,
Patente, Marken & Lizenzen
53839 Troisdorf (DE)**

(56) Entgegenhaltungen:
**EP-A- 0 406 197**
**WO-A-97/02210**
**WO-A-97/21714**
**US-A- 5 679 850**

- **"Encyclopedia of Reagents for Organic Synthesis, vol 5 S.3163-3164" 1995 XP002179702 das ganze Dokument**
- **"Encyclopedia of Reagents for Organic Synthesis, vol 5 S.3160-3161" 1995 XP002179703 das ganze Dokument**
- **LAWSON, D. F. ET AL: "Anionic polymerization of dienes using homogeneous lithium amide (N-Li) initiators" POLYM. PREPR. (AM. CHEM. SOC., DIV. POLYM. CHEM.) (1996), 37(2), 728-729, XP001029218**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 292 599 B1

Printed by Jouve, 75001 PARIS (FR)

## Beschreibung

Die Erfindung betrifft Lösungen von Lithium-Pyrrolidin in Gemischen aus THF und Kohlenwasserstoffen, sowie Verfahren zu ihrer Herstellung und ihre Verwendung.

Lithium-Pyrrolidin gehört zur Familie der Lithium-organoamide, dies sind vielseitig eingesetzte Reagentien in der organischen Synthese, welche sich durch eine hohe Basizität bei geringerer Nucleophilie auszeichnen.

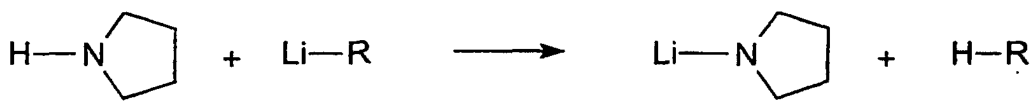

Die Umsetzung kann allgemein in Ethem oder Kohlenwasserstoffen erfolgen, wobei z.B. Methyllithium in Ether oder n-Butyllithium in Hexan verwendet werden (Houben-Weyl, "Methoden der Organischen Chemie", Thieme Verlag, Band XIII/1, 1970; Wakefield, B.J., "The Chemistry of Organilithium Compounds", Pergamon Press, London, 1974; "Organolithium Methods", Academic Press, London, 1988; Brandsma, L. "Preparative Polar Organometallic Chemistry", Vol 1, 1987, und Vol. 2, 1990, Springer-Verlag; M. Schlosser et al, "Organometallics in Synthesis",1994, John Wiley & Sons, Sussex).

Eine weitere Möglichkeit zur Synthese der Li-organoamide besteht in der Ziegler'schen Methode, das Amin mit Lithium in Gegenwart eines Diens umzusetzen:

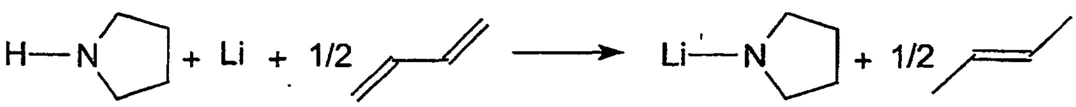

(K.Ziegler, et al., Liebigs Ann. Chem. 511, 64, 1934; Reetz, M.T., et al., Liebigs Ann. Chem., 1471, 1980; WO-97/21714).

Die Li-organoamide sind gewöhnlich feste Substanzen mit pyrophoren Eigenschaften.

Damit sie, vor allem im industriellen Bereich, besser handhabbar sind, werden sie in Lösung in den Handel gebracht; als Lösungsmittel verwendet man Kohlenwasserstoff/Ether-Gemische mit verschiedenen Zusätzen, die einen positiven Einfluß auf die Löslichkeit und Stabilität haben.

Die Löslichkeit in Kohlenwasserstoffen ist oft zu gering, so daß Zusätze wie Lialkoxide verwendet werden (DE 4332652.8).

Die Löslichkeit in Ethem ist im allgemeinen gut, doch zersetzen sich diese Lösungen bei Raumtemperatur, weswegen sie nicht handelsüblich sind. (Leo. A. Paquette, "Encyclopedia of Reagents for Organic Synthesis", Vol 5, 3163, John Wiley, 1995)

Um die Zersetzlichkeit der etherischen Lösungen zu verringern, wird z.B. im Fall des Lithium-di(i-propyl)amids (LDA) ein Lösungsmittelgemisch aus THF und Kohlenwasserstoff verwendet, bei welchem das THF auf einen Gehalt $\leq$ 1,0 mol/mol LDA begrenzt wird (WO-86/03 744).

Ebenso ist es möglich Stabilisatoren, wie Li-Halogenide zuzusetzen (US 5679850).

Eine weitere Möglichkeit der Stabilisierung von z.B. LDA besteht in dem Zusatz von Magnesium-bis(organo) amiden (DE 3905857, US 5320774), was allerdings den Nachteil einer veränderten Reaktivität durch den Zusatz des Erdalkalimetalls bedingt.

Über die besonderen Eigenschaften des Li-Pyrrolidins gibt eine Arbeit von Nudelmann, N,S. et al. Auskunft (J.Chem.Soc, Perkin Trans. 2 (1990), (8), 1461-5), ebenso ist sein spezieller Einsatz in der Pharmasynthese bekannt. (Simvastatin Synthese: US-5,223,415, Merck 1992).

In fester Form liegt das Li-Pyrrolidin als weißes, amorphes Material mit polymerer Leiterstruktur vor, das in Kohlenwasserstoffen unlöslich ist. Bekannt sind die Kristallstrukturen mit den Lewis-Basen PMDETA (Pentamethydiethylenetriamin) und TMEDA (Tetramethylethylendiamin) (R. Snaith et al., J.Chem.Soc., Chem. Commun., 1986, 869; R. Snaith, JACS, 1989, 111, 4719).

Aus Am.Chem.Soc.Div.Polym.Chem, 1996, 37(2), 728-729 (Lawson et al.) und aus der WO 97/21714 sind Lösungen von Lithium-Pyrrolidin in THF und Kohlenwasserstoffen prinzipiell bekannt. Allerdings sind die beschriebenen Lösungen nicht stabil. (Lawson), bzw., es werden keine Angaben über jeweiligen Anteile von Lithium-Pyrrolidin, THF und Kohlenwasserstoffen gemacht (WO 97/21714).

Da sich Lithium-Pyrrolidin wegen seiner besonderen chemischen Eigenschaften deutlich vom LDA abhebt und von besonderem Interesse für die organische Synthese ist, sind stabile, nicht pyrophore Handelsformen wünschenswert, die jedoch bislang nicht bekannt sind. Lithium-Pyrrolidin nimmt innerhalb der Lithium-organoamide eine Sonderstellung ein: Aufgrund seiner starken Basizität ist es besonders reaktiv und greift THF sehr viel stärker an als z.B. LDA. Aufgabe der Erfindung ist es daher, ausreichend konzentrierte, stabile und nicht pyrophore Lösungen von Lithium-Pyrrolidin zu schaffen.

Gelöst wird die Aufgabe durch Lösungen von Lithium-Pyrrolidin in einem Gemisch aus Tetrahydrofuran (THF) und Kohlenwasserstoffen, wobei die Lithium-Pyrrolidin-Konzentration 2 bis 30 Gew.-%, bevorzugt 5 bis 25 Gew.-%, beträgt und wobei das molare Verhältnis Li-Pyrrolidin : THF = 1 : 0,5 bis 1 : 1,5 ist und wobei als Kohlenwasserstoffe aliphatische Kohlenwasserstoffe (cyclische oder acyclische) mit 5 bis 12 C-Atomen oder aromatische Kohlenwasserstoffe mit 6 bis 12 C-Atomen eingesetzt werden.

Obwohl in der von L.A. Paquette herausgegebenen Encyclopedia of Reagents for Organic Syntheses, Vol. 5, auf Seite 3163 offenbart ist, daß Lösungen von Lithium-Pyrrolidin in THF bei Raumtemperatur instabil sind und nach kurzer Zeit LiH bilden, wurde gefunden, daß Lithium-Pyrrolidin im erfindungsgemäßen molaren Verhältnis zu THF ein ganz besonderes Komplexierungsverhalten zeigt und daß dieses Komplexierungsverhalten sowohl die Löslichkeit in Kohlenwasserstoffen als auch die Stabilität dieser Lösungen hinsichtlich Kristallisation bei Abkühlung und thermischer Zersetzung bei Erwärmung bestimmt. Aufgrund IR-spektroskopischer Befunde und kalorimetrischer Daten konnte gezeigt werden, dass gerade im erfindungsgemäßen Konzentrationsbereich der Angriff von Lithium-Pyrrolidin auf THF weitgehend unterbleibt, die THF-Komplexierung begünstigt ist und die Komplexierung einen positiven Einfluss auf die Stabilität und die Löslichkeit bewirkt. Weiter zeigte sich, daß sich die erfindungsgemäßen Komplexe mit zunehmendem molaren Anteil von THF in Kohlenwasserstoffen lösen (siehe Tabelle 1).

Tabelle 1:

| Löslichkeit von Lithium-Pyrrolidin in verschiedenen THF/Kohlenwasserstoffgemischen (Zahlenangaben in Gew.-% Li-Py) | | |
|---|---|---|
| Kohlenwasserstoff | Li-Py : THF - Verhältnis 1 : 0,5 | Li-Py : THF -Verhältnis 1 : 1,5 |
| Hexan | 7 % | 18 % |
| Cyclohexan | 12 % | 23 % |
| Toluol | 13 % | 24 % |

Weiter zeigte sich, daß die Lösungen in diesem Bereich auch hinreichend lagerstabil sind. So hat eine 17 %ige Lösung von Li-Py in THF (ohne Kohlenwasserstoff) eine Zersetzungsrate von k = - 0,9 % Li-Py/Tag bei 0°C und k = - 4,5 % Li-Py/Tag bei 26°C. Im Gegensatz dazu erweist sich eine 17 %-ige Lösung der Zusammensetzung 1 Li-Py : 1,2 THF in Toluol im Bereich von 0°C bis + 40°C als deutlich stabiler (siehe Tabelle 2).

Tabelle 2:

| Zersetzungsraten einer 17 %-igen Li-Py Lösung in THF/Toluol mit einem Li-Py/THF-Verhältnis von 1 : 1,2 | |
|---|---|
| Temperatur | Zersetzungsrate Li-Py : THF 1 : 1,2 in Toluol |
| 0 °C | k = stabil |
| 20 °C | k = stabil |
| 40 °C | k = - 1 % Li-Py pro Tag |

Für die Bestimmung der Stabilität wurde eine Zersetzungs-Reaktion 1. Ordnung angenommen, für diese gilt:

$$k = \ln [\text{Endkonzentration}_{(\text{Aktivbase})} / \text{Anfangskonzentration}_{(\text{Aktivbase})}] \times 100/\text{Lagerzeit in Tagen.}$$

Die Aktivbase wurde nach einer modifizierten Watson-Eastham-Methode bestimmt (S.C.Watson, J.F. Eastham, J. Organomet. Chem. 9, 165, 1967; L.Duhamel, J-C.Plaquevant, JOC, 44, 3404, 1979). Das Verhältnis Li-Py : THF wurde mittels 1-H-NMR-Spektroskopie ermittelt.

Die erfindungsgemäßen Lösungen von Li-Pyrrolidin in einem Gemisch aus THF und Kohlenwasserstoffen

mit dem angegebenen Mischungsverhältnis haben ein solch gutes Lösungs- und Stabilitätsverhalten, dass sie für den industriellen Einsatz geeignet sind.

Die Synthese der erfindungsgemäßen Lösungen kann auf unterschiedlichen Wegen erfolgen:

Zum einen kann metallisches Lithium nach der Ziegler'schen Methode in Gegenwart entsprechender Mengen Pyrrolidin und THF in einem Kohlenwasserstoff mit einem Dien umgesetzt wird. Das Lithium kann dabei als Pulver oder Granulat in Kohlenwasserstoff eingesetzt werden. Die Einsatzmengen von Li, Pyrrolidin, THF und Kohlenwasserstoff richten sich nach der gewünschten Li-Pyrrolidin-Konzentration und nach dem gewünschten Li-Pyrrolidin - THF-Verhältnis der erhaltenen Lösung. Dieses Verhältnis beeinflusst wiederum die gewünschte Löslichkeit und Stabilität. Als Dien wird bevorzugt Isopren eingesetzt, der Einsatz von Styrol und anderen Dienen ist aber auch möglich. Die Reaktion ist exotherm; um thermische Zersetzung zu vermeiden, wird bevorzugt bei 0° bis 20°C gearbeitet, wobei die Wärme über die Dosiergeschwindigkeit geregelt werden kann.

Zum anderen kann aber auch das Pyrrolidin in einem Kohlenwasserstoff mit einem Lithium-organyl wie n-Butyllithium zum Li-Pyrrolidin umgesetzt werden. Das aus dieser Lösung ausfallende Li-Pyrrolidin wird dann mit einer dem gewünschten Verhältnis Li-Pyrrolidin zu THF entsprechenden Menge THF in dem Kohlenwasserstoff in Lösung gebracht.

Verwendung finden die erfindungsgemäßen Lösungen in der organisch-chemischen Synthese und als Polymerisationskatalysator.

Die Erfindung wird im folgenden anhand von Beispielen näher erläutert.

**Beispiel 1:**

10,4 g (1,5 mol) Li-Granulat wurden in 90,1 g (1,25 mol) THF und 71,1 g (1 mol) Pyrrolidin bei 20°C vorgelegt. Dazu wurde innerhalb von 2 Stunden ein Gemisch aus 34,1 g (0,5 mol) Isopren und 200 ml Toluol gleichmäßig zudosiert und die entstehende Wärme durch Mantelkühlung abgeführt. Nach Abklingen der Reaktion wurde vom überschüssigen Li-Metall abfiltriert. Erhalten wurden 380 g Lösung mit einem Aktivbasegehalt von 16,6 % Li-Pyrrolidin, was einer Ausbeute von 82 % entspricht.

**Beispiel 2:**

10,4 g (1,5 mol) Li-Granulat wurden in 90,1 g (1,25 mol) THF und 71,1 g (1 mol) Pyrrolidin in 200 ml Cyclohexan bei 20°C vorgelegt. Innerhalb von 3 Stunden wurden 34,1 g (0,5 mol) Isopren zudosiert. Nach einer 1-stündigen Nachreaktion wurde die Lösung filtriert. Erhalten wurden 350 g Lösung mit einem Aktivbasegehalt von 17,1 %, was einer Ausbeute von 78% entspricht.

**Beispiel 3:**

8 g (1,15 mol) Li-Granulat wurden mit 73,8 g (1,3 mol) THF und 71,1 g (1 mol) Pyrrolidin in 850 ml Hexan bei 20°C vorgelegt und innerhalb von 3 Stunden mit 34,1 g (0,5 mol) Isopren umgesetzt. Nach einer 1-stündigen Nachreaktion wurde die Lösung filtriert. Erhalten wurden 730 g klare Lösung mit einem Aktivbasegehalt von 8,5 %, was einer Ausbeute von 80 % entspricht. Beim Abkühlen fielen aus dieser Lösung 61 g Kristalle aus, deren Zusammensetzung mit 1 Li-Py x 0,6 THF bestimmt wurde.

Die Löslichkeit der Kristalle ergab sich in Hexan mit 7 Gew.-%, in Cyclohexan mit 14,4 Gew.-% und in Toluol mit 13,5 Gew.-%. Durch Zusatz von THF bis zum Verhältnis 1 Li-Py : 1,5 THF erhöhte sich die Löslichkeit in Hexan auf 18,8 Gew.-%, in Cyclohexan auf 23,5 Gew.-% und in Toluol auf 24,0 Gew.-%.

**Beispiel 4:**

90 g einer 18 %-igen n-Butyllithiumlösung in Toluol (entspricht 250 mmol n-BuLi) wurden innerhalb von 30 Minuten bei Raumtemperatur unter Gegenkühlung mit 17,8 g (entspricht 250 mmol) Pyrrolidin umgesetzt, es bildete sich ein farbloser Niederschlag. Durch Zusatz von 21,6 g (300 mmol) THF wurde das gebildete Li-Pyrrolidin in Lösung gebracht. Erhalten wurde eine 17,1 %-ige Lösung von Li-Pyrrolidin (2,22 mmol Aktivbase pro g Lösung) in THF/Toluol mit einem molaren Li-Pyrrolidin/THF-verhältnis von 1 : 1,2. Mit dieser Lösung wurde eine Stabilitätsuntersuchung in einem geschlossen Glasgefäß in einem temperierten Ölbad durchgeführt (Beispiel 5).

**Beispiel 5: Stabilitätsuntersuchung**

Die Lösung aus Beispiel 4 wurde auf ihre Stabilität hin untersucht (Tabelle 3: Veränderung der Aktivbase nach 16 Tagen Lagerzeit bei verschiedenen Temperaturen).

| Lagerzeit / Tage | Temperatur / °C | Aktivbase / mmol/g |
|---|---|---|
| 0 | | 2,22 |
| 16 | 0 | 2,22 |
| 16 | 20 | 2,21 |
| 16 | 40 | 1,91 |

**Vergleichsbeispiel: Stabilität von Lithium-Pyrrolidin in THF ohne Kohlenwasserstoff**

6,9 g (1 mol) Li-Pulver wurden in 300 ml THF und 71,1 g (1 mol) Pyrrolidin bei 20°C innerhalb von 3 Stunden mit 34,1 g (0,5 mol) Isopren umgesetzt und nach 1-stündiger Nachreaktion filtriert. Erhalten wurden 325 g Lösung mit einem Aktivbasegehalt von 17,1 % (Ausbeute. 82 %). Diese Lösung wies nach 14-tägiger Lagerzeit bei 20 °C nur noch einen Aktivbasegehalt von 1,70 mmol/g auf.

## Patentansprüche

1. Lösungen von Lithium-Pyrrolidin in einem Gemisch aus Tetrahydrofuran (THF) und Kohlenwasserstoffen, wobei die Lithium-Pyrrolidin-Konzentration 2 bis 30 Gew.-% beträgt und das molare Verhältnis Li-Pyrrolidin : THF = 1 : 0,5 bis 1 : 1,5 ist und wobei als Kohlenwasserstoffe aliphatische Kohlenwasserstoffe (cyclische oder acyclische) mit 5 bis 12 C-Atomen oder aromatische Kohlenwasserstoffe mit 6 bis 12 C-Atomen eingesetzt werden.

2. Lösungen nach Anspruch 1, **dadurch gekennzeichnet, dass** die Lithium-Pyrrolidin-Konzentration 5 bis 25 Gew.-% beträgt.

3. Lösungen nach Anspruch 2, **dadurch gekennzeichnet, dass** als Kohlenwasserstoffe eine oder mehrere der Verbindungen Pentan, Hexan, Heptan, Octan, Cyclohexan, Tetralin, Toluol, Xylol, Cumol oder Ethylbenzol eingesetzt werden.

4. Verfahren zur Herstellung einer Lösung nach den Ansprüchen 1 bis 3, **dadurch gekennzeichnet, dass** metallisches Lithium in Gegenwart von Pyrrolidin und THF in einem Kohlenwasserstoff mit einem Dien umgesetzt wird.

5. Verfahren nach Anspruch 4, **dadurch gekennzeichnet, dass** das Lithium als Pulver oder als Granulat eingesetzt wird.

6. Verfahren nach einem der Ansprüche 4 oder 5, **dadurch gekennzeichnet, dass** als Dien Butadien oder eines seiner Abkömmlinge oder Styrol oder Naphthalin eingesetzt wird.

7. Verfahren nach Anspruch 6, **dadurch gekennzeichnet, dass** als Dien Isopren eingesetzt wird.

8. Verfahren zur Herstellung einer Lösung nach den Ansprüchen 1 bis 3, **dadurch gekennzeichnet, dass** Pyrrolidin in einem Kohlenwasserstoff mit einem Lithium-organyl zu Li-Pyrrolidin umgesetzt wird und das aus dieser Lösung ausfallende Li-Pyrrolidin mit einer dem gewünschten Verhältnis Li-Pyrrolidin zu THF entsprechenden Menge THF in dem Kohlenwasserstoff in Lösung gebracht wird.

9. Verfahren nach einem der Ansprüche 4 bis 8, **dadurch gekennzeichnet, dass** die Temperatur der Reaktion bevorzugt bei 0° bis 20°C gehalten wird.

10. Verwendung der Lösungen nach den Ansprüchen 1 bis 3 zur organisch-chemischen Synthese und als Polymerisationskatalysator.

## Claims

1. Solutions of lithium pyrrolidine in a mixture of tetrahydrofuran (THF) and hydrocarbons, wherein the lithium pyrrolidine concentration amounts to 2 to 30 % by weight and the molar ratio of Li-pyrrolidine : THF = 1 : 0.5 to 1 : 1.5 and wherein aliphatic hydrocarbons (cyclic or acyclic) with 5 to 12 C-atoms or aromatic hydrocarbons with 6 to 12 C-atoms are used as the hydrocarbons.

**2.** Solutions according to claim 1, **characterised in that** the lithium pyrrolidine concentration amounts to 5 to 25 % by weight.

**3.** Solutions according to claim 2, **characterised in that** one or more of the compounds pentane, hexane, heptane, octane, cyclohexane, tetraline, toluene, xylene, cumene or ethyl benzene is or are used as the hydrocarbons.

**4.** Method for preparing a solution according to claims 1 to 3, **characterised in that** metallic lithium is reacted in the presence of pyrrolidine and THF in a hydrocarbon with a diene.

**5.** Method according to claim 4, **characterised in that** the lithium is used as a powder or as a granulated material.

**6.** Method according to one of claims 4 or 5, **characterised in that** butadiene or one of its derivatives or styrene or naphthalene is used as the diene.

**7.** Method according to claim 6, **characterised in that** isoprene is used as the diene.

**8.** Method for preparing a solution according to claims 1 to 3, **characterised in that** pyrrolidine is reacted with a lithium-organyl in a hydrocarbon to form Li-pyrrolidine, and the Li-pyrrolidine precipitating from this solution is dissolved with a quantity of THF, corresponding to the desired ratio of Li-pyrrolidine to THF, in the hydrocarbon.

**9.** Method according to one of claims 4 to 8, **characterised in that** the temperature of the reaction is preferably held at 0° to 20°C.

**10.** Use of the solutions according to claims 1 to 3 for organic-chemical synthesis and as a polymerization catalyst.

## Revendications

**1.** Solutions de pyrrolidinyl-lithium dans un mélange de tétrahydrofurane (THF) et d'hydrocarbures, dans lesquelles la concentration de pyrrolidinyl-lithium vaut de 2 à 30 % en poids et le rapport molaire (pyrrolidinyl-lithium)/THF vaut de 1/0,5 à 1/1,5, et dans lesquelles on emploie, en tant qu'hydrocarbures, des hydrocarbures aliphatiques, cycliques ou acycliques, comportant de 5 à 12 atomes de carbone ou des hydrocarbures aromatiques comportant de 6 à 12 tomes de carbone.

**2.** Solutions conformes à la revendication 1, **caractérisées en ce que** la concentration de pyrrolidinyl-lithium vaut de 5 à 25 % en poids.

**3.** Solutions conformes à la revendication 2, **caractérisées en ce que** l'on emploie, en tant qu'hydrocarbures, un ou plusieurs des composés suivants : pentane, hexane, heptane, octane, cyclohexane, tétraline, toluène, xylène, cumène et éthylbenzène.

**4.** Procédé permettant de préparer une solution conforme à l'une des revendications 1 à 3, **caractérisé en ce que** l'on fait réagir du lithium métallique avec un diène, en présence de pyrrolidine et de THF, dans un hydrocarbure.

**5.** Procédé conforme à la revendication 4, **caractérisé en ce que** l'on emploie du lithium qui se trouve en l'état de poudre ou de granulat.

**6.** Procédé conforme à l'une des revendications 4 et 5, **caractérisé en ce que** le diène qu'on emploie est du butadiène ou de l'un de ses dérivés, du styrène ou du naphtalène.

**7.** Procédé conforme à la revendication 6, **caractérisé en ce que** le diène qu'on emploie est de l'isoprène.

**8.** Procédé permettant de préparer une solution conforme à l'une des revendications 1 à 3, **caractérisé en ce que** l'on fait réagir de la pyrrolidine avec un organo-lithien, dans un hydrocarbure, pour obtenir du pyrrolidinyl-lithium,

et **en ce que** l'on fait passer à nouveau en solution dans l'hydrocarbure ce pyrrolidinyl-lithium, qui a précipité, au moyen de la quantité de THF qui fournit le rapport (pyrrolidinyl-lithium)/THF voulu.

**9.** Procédé conforme à l'une des revendications 4 à 8, **caractérisé en ce qu'**on maintient la température de réaction, de préférence, à une valeur de 0 à 20 °C.

**10.** Emploi de solutions conformes à l'une des revendications 1 à 3 en synthèse organique et comme catalyseurs de polymérisation.